# EUROPEAN PATENT APPLICATION

(11) **EP 1 707 210 A1**
(43) Date of publication of application: **04.10.2006**
(21) Application number: 04816711.8
(22) Date of filing: 30.12.2004
(51) Int. Cl.: A61K 35/64, A61P 31/12

(54) **ANTIVIRAL PREPARATION**

(30) Priority: 15.01.2004 RU 2004100857
(71) Applicant: Chernysh, Sergey Ivanovich, St. Petersburg (RU)
(72) Inventor: Chernysh, Sergey Ivanovich, St. Petersburg (RU)
(74) Representative: Valea AB
(86) International application number: PCT/RU2004/000542
(87) International publication number: WO 2005/067949

(57) **Abstract**

The aim of the invention is to extend a range of antiviral drugs by using entovirons in the form of components of an insect hemolymph exhibiting a direct antiviral action. The entovirons obtainable from Odonata, Coleoptera, Lepidoptera and Diptera representatives are most efficient in the form of drug prototypes.

## Description

### Field of the Invention

The present invention relates to natural pharmaceutically active compounds, and can be used to obtain antiviral medications and to treat viral infections.

### Background of the Invention

Known are pharmaceutical that include complex of extractive substances from animal and vegetative tissues, which are used for treatment of viral infections by immnnopotentiation and direct impact on virions or virus infected cells.

The method for producing proteins from CD4-positive T-lyrnphocytes and bone marrow cells, which proteins suppress development of human immunodeficiency virus, is known [US pat. 5,480,782] (1).

The external drug that includes extract of *Ginkgo biloba* and possesses antiviral and antibacterial activity, is known [DE pat. 4334600 A1] (2).

The patent WO 81/03124 [3] discloses polypeptide fraction extracted from *Mytilus edulis* mollusk, which fraction is active against various viruses, bacteria and protozoa.

Known are phytogenic antiviral drugs, which action mechanism is based on inactivation of free viral particles, blockage of their transport through cell membrane, or suppression of virus replication in host cell [Ershov F.I. Antiviral preparations, Moscow, Medicina, 1998] (4).

Known is the multi-component composition obtained from tissues of *Calliphora vicina* Robineau-Desvoidy (Diptera, Calliphoridae) insect and exhibiting-an antiviral activity [US pat. 6,337,093] (5). From this composition, the peptide alloferon has been obtained, which possesses antiviral and antitumor activity [RU 2172322] (6). Antiviral activity of said composition and alloferon as its reactant is caused by immunopotentiating properties: ability to induce interferon synthesis and to enhance cytotoxic activity of natural killers [Chernysh et al. Antiviral and antitumor peptides from insects // PNAS, 2002, 99, p. 12628-12632] (7). At the same time, neither said composition nor alloferon provide direct protective influence upon cells subjected to viral infection.

Said composition and alloferon as its reactant is the only antiviral agent obtained from insects known at present. Under this attribute, they are the closest analogs of the present invention.

Natural pharmaceuticals mentioned above and natural analogs thereof extend the store of antiviral agents. However, pharmaceuticals known from the art do not cover existing needs, and in this connection, viral infections are still the most dangerous and intractable group of human and animal diseases.

So, the object of the present invention is in extending the store of antiviral pharmaceuticals.

### Summary of the Invention

While creating the present invention, it has been discovered that hemolymph of various insects comprises extractive substances, which increase, when being introduced into mammal cells, viral infection resistance of said cell culture. Further systematic study of pterygots (class Insecta, subclass Pterigota) allowed to disclose that analogous antiviral activity is present in several taxonomic ranks: species, genera, families and orders. As a result, a methodology for search of antiviral medications, which are included in insect tissues, has been elaborated. Basing upon said methodology, species and groups of higher rank, which are the most perspective as source of antiviral substances, have been determined.

Thus, the subject-matter of the present invention relates to the novel group of antiviral medications, direct action antiviral medications, obtained by extraction from insect tissues, particularly from representatives of orders of Odonata (dragonflies), Mantoptera (soothsayers), Hemiptera (hemipterans), Coleoptera (beetles), Diptera (two-winged flies), Mecoptera (scorpion flies) and Lepidoptera (scale-winged insects).

Medications of direct action, in the case, implicate individual substances or mixtures thereof, which increase resistance of organism cells towards viral infection by destruction of virus, blockage of replication thereof or by other way, which can run aside from immune system. Distinctive and generic feature of such medications is the increase of target cell resistance towards viral attack outside the organism (in vitro), e.g. when medication is introduced into cultural media with cells. Within the context of the present invention, the antonym for direct action medications are immunomodulators, which pharmaceutical activity is based upon stimulation of organism immune response. It is obvious that some insect metabolites can possess properties of antiviral medication with both direct and immunomodulating action mechanism. The latter fact should not prevent these materials to be included in claimed group of medications, provided that therapeutic efficacy of said substances is caused completely or partially by direct antiviral action.

It is also obvious that antiviral substances Primarily obtained from insect tissues can be reproduced by means of chemical or biological synthesis, and are used in the same or modified form as medications. Basic structure can be modified in order to increase stability, bioavailability, and antiviral activity, and to decrease toxicity or other side effects of medication. Individual substances thus obtained or mixtures thereof can be used as a part of pharmaceutical compositions and in combination with other antiviral drugs.

Above-mentioned set of direct action antiviral substances obtained from insect tissues is proposed to be called entovirones ("ento-" from Greek "entomon" - insect, and "-viron" from Latin "virus").

In order to study entovirones distribution within class of insects, hemolymph samples from 31 insect species were studied within one technique:
Mantoptera order: *Iris polystictica*
Hemiptera order: *Aphrophora salicina*
Coleoptera order: *Pseudophonus rufipes, Acanthocinuss aedilis, Nicrophorus vespillo*
Hymenoptera order: *Vespa crabro, Diprion pini, Athalia colibri*
Diptera order: *Delia brassicae, Calliphora uralensis, Musca domestica, Epysyrphus balteatus, Stratiomys singularior, Gasterophilus veterinus, Lunatipula sublunata*
Mecoptera order: *Panorpa communis*
Lepidoptera order: *Mamestra persicaria, Diachrysia chrysitis, Operophtera brumata, Cossus cossus, Dendrolimus sibiricus, Lasiocampa trifoli, Axylia putris, Notodonta dromedarius, Sphinx ligustri, Ephestia kuhniella, Tortrix viridana*
Odonata order: *Aeschna cyanea, Aeschna grandis, Libellula quadrimaculata, Somatochlora metallica*

Presence of entovirones has been established in samples from 26 species, which relate to all of orders of insects under investigation (Table 1). Only 5 samples have displayed the negative result.

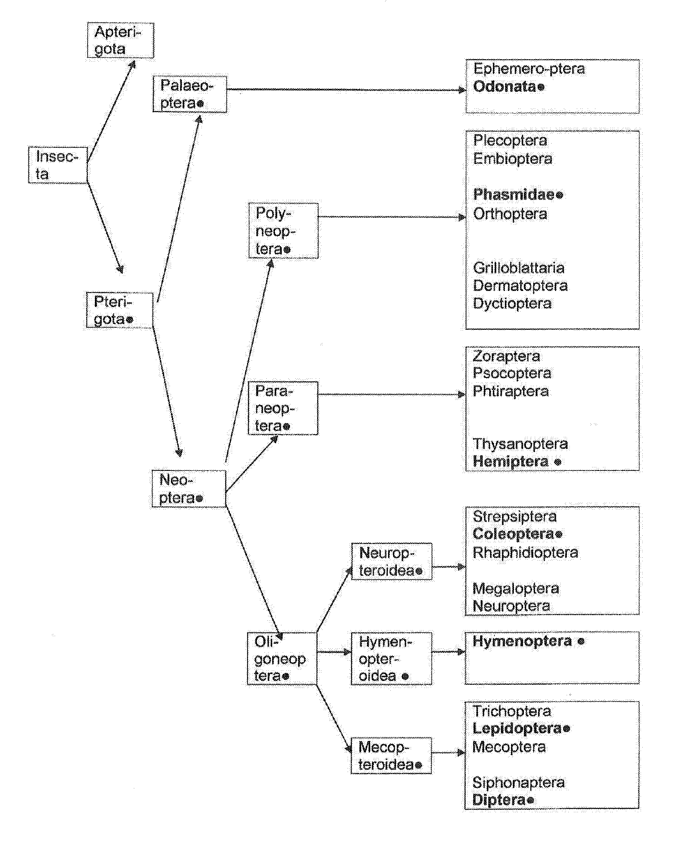

Investigated orders, as one can see from Fig. 1, relate to two main branches of subclass Pterigota (pterygots) evolution: Palaeoptera and Neoptera. On this basis, entovirons can be considered to be the specific feature of the whole Pterigota subclass. This conclusion is confirmed by the fact that entovirons have been found in all subdivisions of Neoptera group, including Polyneoptera, Paraneoptera and Oligoneoptera, as well as in all subdivisions of the most plentiful Oligoneoptera group, namely Neuropteroidea, Hymenopteroidea and Mecopteroidea. Representatives of Apterigota subclass were not included in this research because of technical difficulties of getting the hemolymph samples. Thus, claim of the present invention is limited to representatives of Pterigota subclass.

Although entovirons are widespread throughout insects, their activity differs substantially when compared with representatives of various groups. Most active are entovirons of Odonata (dragonflies) order, particularly of dragonflies from Aeschnidae family like *Aeschna grandis* and *Aeschna cyanea* as well as from Libellulidae particularly *Libellula quadrimaculata* and *Somatochlora metallica.* Another group possessing high entovirons activity is a part of Lepidoptera order Noctuidae family represented by *Diachrysia chrysitis* and *Mamestra persicaria* species of the said family, as well as of Geometridae family (e.g. *Operophtera brumata*). Moreover, high entovirons activity is typical for several representatives of Coleoptera (*Pseudophonus rufipes,* Carabidae) and Diptera (*Stratiomys singularior,* Stratiomyidae) orders. Representatives of said species, families and orders are the most effective sources of entovirons.

### Detailed Descriptions of the Invention

### EXAMPLE 1. Entoviron extraction from insects hemolymph.

Initial experimental biological material (insect larvae of certain order and development stage) were obtained from natural populations. After determining the species of each insect, they were kept in laboratory at ambient temperature and natural humidity. Immune system of insects was activated by puncturing cuticle with a needle wetted with a suspension of heat-killed (1 min in boiling water bath) Escherichia coli and Micrococcus luteus bacteria (10⁹ cells of each type per 1 ml). Thus immunized larvae were incubated at ambient temperature for 24 hours. After that the surface of larvae was sterilized with 70 % ethanol, next larvae were washed by sterilized distilled water and predried at filter paper. Hemolymph of larvae was collected through cuticle incision into ice-cooled test-tubes containing aprotinine and phenylthiourea in order to suppress proteolytic and phenoloxidase activity of hemolymph. Obtained samples were immediately frozen in liquid nitrogen and stored before use at -20 °C.

Unfrozen hemolymph was centrifuged at 4000 g for 10 min and supernatant was acidified by trifluoroacetic acid until acid concentration reached 0.05 % (v/v). Thus obtained sample was deposited on Sep-Pak column with C₁₈ sorbent (Waters company) on the basis of 3 ml of hemolymph per 1 g of sorbent. The column was washed with 0.05 % trifluoroacetic acid to remove hydrophilic components of hemolymph. After that adsorbed hydrophobic components were eluted by 50 % acetonitrile in 0.05 % trifluoroacetic acid. Thus obtained mixture of hydrophobic components was freeze-dried and used for further antiviral activity studies.

### EXAMPLE 2. Antiviral activity of entovirons separated from insects hemolymph.

Antiviral activity was determined *in vitro* using common system for interferon antiviral activity determination (Chernysh et al. Antiviral and antitumor peptides from insects // PNAS, 2002, 99, p. 12628-12632). Human cell culture L41 was incubated in wells of 96-well culture plates until cell monolayer is formed on the bottom of each well. Then the substance under test was placed into wells. Simultaneously vesicular stomatitis virus (VSV, Indiana strain) in a dose equal to 100 CD₅₀ (cytopathogenic dose causing death of 50 % monolayer cells) was added into wells, Equivalent quantity of pure solvent was added to control wells. Cell culture infected by virus was incubated at 37 °C for 18 hours, then cells were visualized by 0.1 % solution of a crystal-purple dye, Percentage of cells destroyed by the virus was estimated using measurements of optical density of extracted dye at the wavelength of 590 nm. Increase of survived monolayer cells percentage compared to controls indicated the activity of entovirons. Obtained results were rated as positive when certainty level for differences between experiment, and control was less than 0,01 (P < 0.01) according to Student's test.

Results of antiviral activity studies for hemolymph samples obtained from 31 insect species are presented in Table 1. Positively rated samples are marked as "•". There were 26 positive rated samples, or 84% of the full experimental set. As one can see from Fig. 1, samples containing entovirons relate to 8 orders, which represent full set of Pterigota subdivisions: Palaeoptera and Neoptera, Polyneoptera, Paraneoptera and Oligoneoptera, Neuropteroidea, Hymenopteroidea and Mecopteroidea, Thus entovirons should be considered to be typical hemolymph component for all insects.

Four sample groups relating to certain orders and families rise above investigated samples due to their high entoviron activity. The first group includes samples obtained from hemolymph of dragonflies (Odonata order), which are represented by Aeschnidae and Libellulidae families (*Aeschna grandis* and *Libellula quadrimaculata,* respectively). The second group includes samples obtained from Coleoptera order, which is represented by Carabidae family (e.g. *Pseudophonus rufipes*). The third group includes hemolymph samples from Lepidoptera, which is represented by Noctuidae family on an example of *Diachrysia chrysitis* and *Mamestra persicaria* noctnids as well as Geometridae family on an example of *Operophtera brumata* geometrid. And the last, fourth group includes samples obtained from Diptera order, represented by Stratiomyidae family on an example of *Stratiomys singularior.*

Listed orders, families and species are the most effective sources of entovirons.

### Best variant of the invention embodiment

Best variant of the invention embodiment is presented in the Example 1 describing a method of entovirons isolation, and Example 2 demonstrating antiviral activity of different representatives of the group of antiviral preparations. According to the data obtained, entoviron of an insect *Libellula quadrimaculata* deinonsttates maximum antiviral activity.

### Industrial applicability

Industrial applicability of the invention is confirmed by the results desclosed in the application and demonstrating that entoviron allows to effectively prevent virus infected cells death, which is a of antiviral therapy. Because of that entovimns could be used for treatment of viral infections. Since industrial technologies of bioactive materials production are well developed for the time being, entovirons manufacturing by chemical or biological methods has no great difficulty.

**Table 1. Antiviral activity of entovirons, obtained from insect hemolymph**

| Specimen | Family | Surviral value for infected cells, % | | Presence of entovirons (P<0.01) |
|---|---|---|---|---|
| | | Series 1 | Series 2 | |
| Control | | 8.2 ± 0.23 | 6.0 ± 0.27 | |
| **Odonata order** | | | | |
| Aeschna cyanea | Aeschnidae | | 17.0± 1.0*** | • |
| Aeschna grandis | Aeschnidae | | 38.5± 3.5*** | • |
| Libellula quadrimaculata | Libellulidae | 69.5± 5.5*** | | • |
| Somatochlora metallica | Libellulidae | | 15.5± 1.5*** | • |
| **Mantoptera order** | | | | |
| Iris polystictica | Mantidae | 13.5 ± 1.5*** | | • |
| **Hemiptera order** | | | | |
| Aphrophora salicina | Aphrophoridae | 11.0 ± 1.0** | | • |
| **Coleoptera order** | | | | |
| Pseudophonus rufipes | Carabidae | 30.0 ± 3.0*** | | • |
| Acanthocinuss aedilis | Cerambicidae | | 12.0 ± 1.0*** | • |
| Nicrophorus vespillo | Silphidae | | 9.0 ± 0.0*** | • |
| **Diptera order** | | | | |
| Delia brassicae | Anthomyidae | | 10.0 ± 0.0*** | • |
| Calliphora uralensis | Calliphoridae | 9.3 ± 1.75 | | |
| Musca domestica | Muscidae | | 8.5 ± 0.5 | |
| Epysyrphus balteatus | Syrphidae | 17.5± 2.5*** | | • |
| Stratiomys singularior | Stratiomyidae | 32.5 ± 4.5*** | | • |
| Gasterophilus veterinus | Gasterophilidae | 11.5 ± 1.5*** | | • |
| Lunatipula sublunata | Tipulidae | | 9.5 ± 1.5** | • |
| **Mecoptera order** | | | | |
| Panorpa communis | Panorpidae | 15.0 ± 2.0*** | | • |
| **Hymenoptera order** | | | | |
| Vespacrabro | Vespidae | 10,1±0,9* | | |
| Diprion pini | Diprionidae | | 7.0± 1.0 | |
| Athalia colibri | Tentridinidae | | 9.5±0.5*** | • |
| **Lepidoptera order** | | | | |
| Mamestrapersicana | Noctuidae | 25.0±2.0*** | | • |
| Diachrysia chrysitis | Noctuidae | 23,5±2.5*** | | • |
| Operophtera brumata | Geometridae | 35.5±3.5*** | | • |
| Cossuscossus | Cossidae | 7.1±1.4 | | |
| Dendrolimus sibiricus | Lasiocampidae | | 12.5±0.5*** | • |
| Lasiocampa trifoli | Lasiocampidae | | 9.5±0.5*** | • |
| Axylia putris | Noctuidae | | 11.5±0.5*** | • |
| Notodonta dromedarius | Notodontidae | | 9.5±0.5*** | • |
| Sphinx ligustri | Sphingidae | | 10.0±1.0*** | • |
| Ephestia kuhniella | Pyralydae | | 10.0±0.0*** | • |
| Tortrix viridana | Tortricidae | 12.5±1.5*** | | • |

| | | | | |
|---|---|---|---|---|
| *P< 0.05; **P< 0.01; ***P<0.001 | | | | |

### Reference

1. US Pat. 5,480,782
2. DE Pat. 4334600 Al
3. WO Pat. 81/03124
4. Ershov F.I., Antiviral preparations, Moscow, Medicine publishing house, 1998
5. US Pat. 6,337,093
6. RU Pat. 2172322
7. Chernysh et al., Antiviral and antitumor peptides from insects, PNAS// 2002, 99, p. 12628-12632

## Claims

1. An antiviral agent increasing survivability of host cells infected by virus, wherein insects Pterigota subclass is used as the source of said antiviral agent.

2. The antiviral agent of claim 1, wherein species of Odonata order are used as insects of Pterigota subclass.

3. The antiviral agent of claim 2, wherein species of Aeschnidae family are used as insects of Odonata order.

4. The antiviral agent of claim 3, wherein dragonflies of *Aeschna* genus are used as insects of Aeschnidae family.

5. The antiviral agent of claim 2, wherein species of Libellulidae family are used as insects of Odonata order.

6. The antiviral agent of claim 5, wherein dragonflies of *Libellula* genus are used as insects of Libellulidae family,

7. The antiviral agent of claim 1, wherein species of Malltoptera, Hemiptera, Mecoptera, Hymenoptera order are used as insects of Pterigota subclass.

8. The antiviral agent of claim 1, wherein species of Coleoptera order are used as insects of Pterigota subclass.

9. The antiviral agent of claim 8, wherein species of Carabidae family are used as insects of Coleoptera order.

10. The antiviral agent of claim 9, wherein carabuses of *Pseudophonus* genus are used as insects of Carabidae family.

11. The antiviral agent of claim 1, wherein species of Diptera order are used as insects of Pterigota subclass.

12. The antiviral agent of claim 11, wherein species of Stratiomyidae family are used as insects of Diptera order.

13. The antiviral agent of claim 12, wherein soldier flies of *Stratiomys* genus are used as insects of Stratiomyidae family.

14. The antiviral agent of claim 1, wherein species of Lepidoptera order are used as insects of Pterigota subclass.

15. The antiviral agent of claim 14, wherein species of Noctuidae family are used as insects of Lepidoptera order.

16. The antiviral agent of claim 15, wherein noctuids of Mamestra genus are used as insects of Noctuidae family.

17. The antiviral agent of claim 15, wherein noctuids of *Diachrisia* genus are used as insects of Noctuidae family.

18. The antiviral agent of claim 14, wherein species of Geometridae family are used as insects of Lepidoptera order.

19. The antiviral agent of claim 18, wherein geometrids of *Operophtera* genus are used as insects of Geometridae family.

20. Individual substances of the composition of antiviral agent of claims 1-19, increasing survivability of host cells infected by virus.

21. Analogs of individual substances of claim 20, obtained by means of chemical or biological synthesis, and derivatives thereof, e.g. salts and amides of corresponding peptides and proteins.

22. Pharmaceutical compositions comprising antiviral agent of claims 1-19 or individual substances of claim 20 thereof, or analogs of individual substances of claim 21.

23. Use of antiviral agent of claims 1-19, or individual substances of claim 20 thereof, or analogs of individual substances of claim 21 for development of antiviral medications.

24. Use of antiviral agent of claims 1-19, or individual substances of claim 20 thereof, or analogs of individual substances of claim 22 for treatment and prophylaxis of viral infections.
